# EUROPÄISCHE PATENTSCHRIFT

(11) **EP 3 254 358 B1**
(45) Veröffentlichungstag und Bekanntmachung des Hinweises auf die Patenterteilung: **30.11.2022**
(21) Anmeldenummer: 16701801.9
(22) Anmeldetag: 28.01.2016
(51) Int. Cl.: H02K 3/47, A61B 17/16

(54) **CHIRURGIEMOTOR**
SURGICAL MOTOR
MOTEUR CHIRURGICAL

(30) Priorität: 02.02.2015 DE 102015101487
(43) Veröffentlichungstag der Anmeldung: 13.12.2017
(73) Patentinhaber: Aesculap AG, 78532 Tuttlingen (DE)
(72) Erfinder: KAHLER, Thomas, 78606 Seitingen-Oberflacht (DE); BARTH, Jürgen, 78588 Denkingen (DE); AUBER, Stephanie, 78532 Tuttlingen (DE); HÖGERLE, Roland-Alois, 78532 Tuttlingen (DE)
(74) Vertreter: Winter, Brandl - Partnerschaft mbB
(86) Internationale Anmeldenummer: PCT/EP2016/051809
(87) Internationale Veröffentlichungsnummer: WO 2016/124478

(56) Entgegenhaltungen:
- EP-A1- 2 135 567
- EP-B1- 2 135 567
- WO-A2-2011/113887
- DE-A1- 4 438 132
- US-A1- 2012 274 167

## Beschreibung

Die Erfindung betrifft einen Elektromotor, insbesondere Hochgeschwindigkeits-Chirurgiemotor, mit einem Rotor, der konzentrisch von einem zumindest eine Statorwicklung tragenden im Wesentlichen hohlzylindrischen Spulenkörper umgeben ist. Die Erfindung betrifft ferner die Herstellung des Spulenkörpers und einer Statorwicklung.

Derartige Motoren zeichnen sich dadurch aus, dass sie bei kleinster Bauweise sehr große Leistungen bereitstellen, so dass ohne weiteres Drehzahlen bis zu 200.000 U/min erreichbar sind. Damit eignen sich solche Motoren insbesondere für den Antrieb chirurgischer Instrumente.

Ein Elektromotor mit einem Rotor, der konzentrisch von einem zumindest eine Statorwicklung tragenden im Wesentlichen hohlzylindrischen Spulenkörper umgeben ist, ist beispielsweise im Dokument EP 2 135 567 A1 beschrieben. In diesem bekannten Fall ist allerdings der Aufbau so, dass die Statorwicklung in eine Vergussmasse eingebettet ist.

Aus dem Dokument DE 44 38 132 A1 ist es bekannt, bei einem Antriebsmotor für eine Spaltrohrpumpe zwischen Statorwicklung und Rotor ein Spaltrohr, insbesondere einen Spalttopf einzusetzen, der die Ständerwicklung trägt.

Im Dokument US 2012 / 274167 A1 ist ein Elektromotor beschrieben, der einen Spulenkörper mit Speichenkörpern und Rippen aufweist. Die Rippen und die Speichenkörper sind dazu ausgebildet, Drähte der Statorwicklung zu fixieren.

Moderne Motoren weisen demgegenüber einen in der Regel im Spritzgussverfahren aus Kunststoff hergestellten Spulenkörper auf, auf dem zumindest eine Statorwicklung aufgebracht wird. Diese Konstruktion ermöglicht es, die zumindest eine Statorwicklung noch platzsparender im Motor unterzubringen. Gleichzeitig können die Herstellungskosten dadurch gesenkt werden, dass der Spulenkörper mit einer immer komplexeren Form spritzgegossen wird, die es ermöglicht, den Spulenkörper im Motorgehäuse und die Spulenwicklung mit vorbestimmter Konfiguration ohne zusätzliche Bauelemente dauerhaft zu fixieren.

Um die Leistung dieser Motoren weiter anzuheben, ist man bemüht, die Luftspalte zwischen dem Rotor und dem Spulenkörper immer weiter zu verringern, beispielsweise in Bereiche zwischen 0,1 und 0,3 mm, wobei die weitere Besonderheit zu berücksichtigen ist, dass die Spulenkörper oftmals eine im Vergleich zum Innendurchmesser erhebliche axiale Länge haben. Es hat sich gezeigt, dass es bei herkömmlichen Motoren dieser Bauart durch plötzliches Blockieren des Rotors zu einem frühzeitigen Ausfall gekommen ist.

Der Erfindung liegt deshalb die Aufgabe zugrunde, einen Elektromotor der vorstehend beschriebenen Art derart weiter zu entwickeln, dass unter Beibehaltung einer einfachen Montage selbst dann, wenn eine verhältnismäßig lange Statorwicklung zum Einsatz kommt, zuverlässig dafür gesorgt ist, dass die Motorleistung über die gesamte Lebensdauer des Motors gleichbleibend hoch bleibt.

Diese Aufgabe wird durch die Merkmale des Anspruchs 1 gelöst.

Erfindungsgemäß wurde erkannt, dass der in der Regel im Spritzgussverfahren mit verhältnismäßig komplexer Geometrie, d.h. mit verschiedenen, räumlich zueinander eng tolerierten Passungsflächen, hergestellte Spulenkörper insbesondere dann, wenn er eine im Vergleich zum Durchmesser verhältnismäßig große axiale Länge hat, bereits bei der Aufbringung der Statorwicklung bzw. bei den darauffolgenden Montageschritten, kleinsten, unkontrollierten Verformungen unterliegt, durch die der Luftspalt über die Länge des Rotors ungleichmäßig wird. Im Laufe des Betriebs des Motors, in dem dieser regelmäßig aufbereitet bzw. einem Sterilisierungsprozess, beispielsweise einer Dampfsterilisierung, unterzogen wird, verstärkt sich diese Ungleichmäßigkeit im Luftspalt immer mehr, bis der Rotor schließlich blockiert.

Erfindungsgemäß wird der Spulenkörper entweder von einem nichtleitenden Vollkeramikkörper oder als Verbund-Körper ausgebildet, bei dem in einen im Spritzgussverfahren hergestellten Kunststoffkörper zumindest ein formstabilisierendes Gerüst-Element durch Umspritzen mit Kunststoff eingebettet ist. Die Verwendung eines Vollkeramik-Körpers ist für einfachere Geometrien sinnvoll, um die Herstellungskosten klein zu halten. Die Ausgestaltung des Spulenkörpers als Verbund-Körper hat den besonderen Vorteil, dass die Herstellungskosten weiterhin klein gehalten werden können und die Formgebung des Spulenkörpers unverändert komplex ausgebildet werden kann. Durch den erfindungsgemäßen Umspritzprozess kommt es zu einer besonders innigen Verbindung zwischen den Verbund-Komponenten, so dass das Gerüst-Element dem Spulenkörper selbst dann, wenn es nur ein sehr kleines Volumen in Anspruch nimmt, eine erheblich gesteigerte Festigkeit gibt, wodurch die oben beschriebenen unkontrollierten Verformungen des Spulenkörpers auch im Langzeitbetrieb und auch bei wiederholter Aufbereitung des Elektromotors nicht mehr auftreten. Durch eine gezielte Oberflächenbehandlung des Gerüst-Elements kann die Verzahnung noch verbessert werden. Es gelingt somit, mit kleinsten Luftspalten zu arbeiten und so eine große Motorleistung über die gesamte Lebensdauer des Motors bereit zu stellen, und zwar selbst dann, wenn die Spulenkörper eine verhältnismäßig große axiale Länge haben.

Die Erfindung betrifft einen Elektromotor, insbesondere Hochgeschwindigkeits-Chirurgiemotor, mit einem Rotor, der konzentrisch von einem zumindest eine Statorwicklung tragenden im Wesentlichen hohlzylindrischen Spulenkörper umgeben ist. Der Spulenkörper und die Statorwicklung bilden dabei einen Stator und der Spulenkörper ist auf seiner Außenseite mit gleichmäßig verteilten Speichenkörpern und Rippen ausgestattet, die zum stirnseitigen Ende hin in Rippenfortsätzen auslaufen, wobei die Speichenkörper, Rippen und Rippenfortsätze dafür vorgesehen und ausgebildet sind, Drähte der Statorwicklung in genau strukturierter Form zu fixieren. Zur Bereitstellung des Stators werden also
- zunächst der Spulenkörper mit pilzartig ausgestalteten Speichenkörpern und Rippenfortsätzen, die sich zum stirnseitigen Ende hin radial leicht verjüngen, als Verbund-Körper ausgebildet wird, indem zumindest ein formstabilisierendes Gerüst-Element in einen im Spritzgussverfahren hergestellten Kunststoffkörper durch Umspritzen mit Kunststoff eingebettet wird und
- anschließend die Drähte auf und um die Rippen und Rippenfortsätze gewickelt.

Es hat sich herausgestellt, dass bereits dann, wenn das Gerüst-Element die einfache Form eines Hülsenkörpers hat, genügend Zusatz-Festigkeit bereitgestellt wird, um die Langzeit-Funktionszuverlässigkeit des Motors bereitzustellen.

Vorzugsweise besteht der Hülsenkörper aus nichtleitendem Material, wobei er zumindest abschnittsweise eine zylindrische Innenoberfläche des Spulenkörpers ausbildet. Mit dieser Ausgestaltung wird die Formstabilität des Spulenkörpers an den entscheidenden Stellen sichergestellt, d.h. dort, wo es auf die dauerhafte Einhaltung des Luftspalts zum Rotor ankommt.

Es hat sich in Versuchen herausgestellt, dass es zu einer ausreichenden Erhöhung der Formstabilität schon genügt, wenn der Hülsenkörper mit einer Wandstärke ausgebildet wird, die im Bereich von 0,01 bis 0,03 x D liegt, wobei D den Innendurchmesser des Spulenkörpers bedeutet. Auf diese Weise bleibt die Gesamtkonstruktion des Spulenkörpers von der Modifikation weitgehend unbeeinflusst.

Chirurgie-Elektromotoren arbeiten mit Rotoren, die einen Außendurchmesser von etwa 12 mm haben. In diesem Fall kann die Wandstärke des Hülsenkörpers im Bereich zwischen 0,1 bis 0,5 mm liegen.

Wenn das Gerüst-Element von einer Keramikkomponente, insbesondere einer Oxidkeramik-Komponente gebildet ist, kann es dem Rotor direkt benachbart sein, so dass es radial innen nicht mit Kunststoff umspritzt werden muss. Außerdem kann der Keramik- Komponente mit einfachen Verfahrensschritten eine Oberfläche verliehen werden, die sich gut für eine Verzahnung mit dem Kunststoff eignet. Der Innendurchmesser der Keramikhülse kann mit vertretbarem Aufwand auf das entsprechend genaue Maß bearbeitet werden, um selbst kleinste Luftspalte zu realisieren. Derartige Keramikwerkstoffe haben darüber hinaus den Vorteil, dass sie ohne weiteres mit Heißdampf sterilisierbar sind.

Vorteilhafte Weiterbildungen sind Gegenstand der Unteransprüche.

Nachstehend wir anhand schematischer Zeichnungen ein Ausführungsbespiel der Erfindung näher erläutert. Es zeigen:
Figur 1 eine Stirnansicht eines Spulenkörpers zur Aufnahme einer Statorwicklung eines Chirurgie-Elektromotors;
Figur 2 in vergrößerter Darstellung die Einzelheit "II" in Figur 1;
Figur 3 die Schnittansicht "III-III" gemäß Figur 1;
Figur 4 die Schnittansicht "IV-IV" in Figur 3; und
Figur 5 eine perspektivische Ansicht der auf dem Spulenkörper aufgebrachten Statorwicklung.

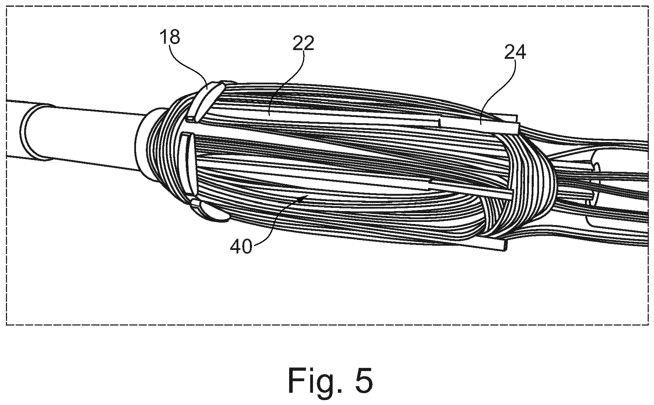

In den Figuren ist mit dem Bezugszeichen 10 der Spulenkörper eines nicht näher dargestellten Elektromotors bezeichnet, der beispielsweise als Hochgeschwindigkeits-Chirurgiemotor ausgebildet sein kann. Der im Wesentlichen hohlzylindrisch ausgebildete Spulenkörper 10, der zumindest eine - wie in Figur 5 dargestellte - Statorwicklung 40 trägt, umgibt einen - nicht näher dargestellten - Rotor, der in der Regel einen zylindrischen Permanentmagneten trägt. Der Rotor ist also konzentrisch von dem im Wesentlichen hohlzylindrischen Spulenkörper 10 umgeben, wobei ein durchgängiger Luftspalt aufrecht erhalten ist, der im zehntel-mm-Bereich liegt, um Leistungsverluste des Motors zu minimieren.

Solche Motoren haben eine kleine Baugröße. Beispielsweise beträgt der Durchmesser des Rotors etwa 12 mm, wobei die Länge L10 des Spulenkörpers im Bereich von etwa 40 mm liegt.

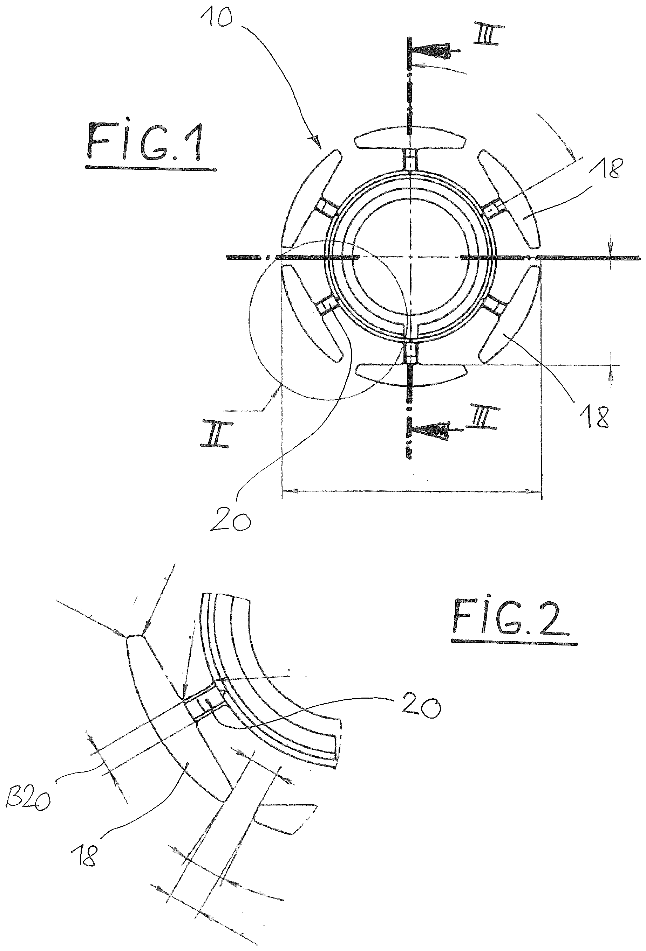

Weil dem Spulenkörper zur Minimierung der Baugröße des Motors neben der Fixierung der Statorwicklung - wie aus Figur 5 ersichtlich - noch weitere Funktionen, wie z.B. die Stabilisierung im Motorgehäuse übertragen werden, erhält der Spulenkörper 10 - wie in den Figuren 1 bis 5 gezeigt - eine verhältnismäßig komplexe Form, so dass er in der Regel als Präzisions-Kunststoff-Spritzgussteil ausgeführt ist. Hierbei können alle bekannten Kunststoffarten Verwendung finden, die sich für diese Art der Herstellung eignen. Beispiele für solche Kunststoffe sind vorzugsweise hochtemperaturbeständige thermoplastische Kunststoffe wie Polyetheretherketon (PEEK) aus der Stoffgruppe der Polyaryletherketone oder Polyphenylensulfid (PPS).

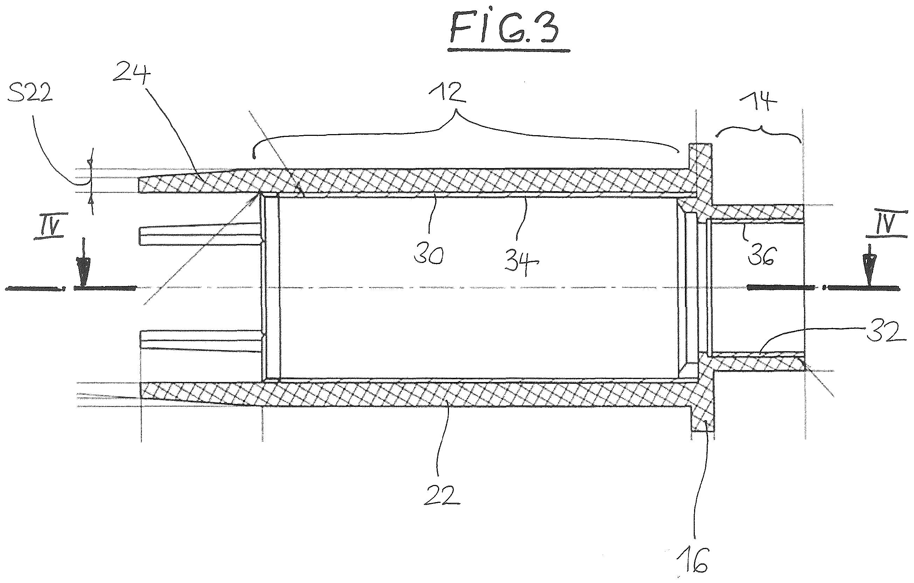

Der Spulenkörper 10 der gezeigten Ausführungsform hat im Wesentlichen die Form einer gestuften zylindrischen Hülse, mit einem ersten längeren Hülsenabschnitt 12 und einem zweiten kürzeren Hülsenabschnitt 14. Zwischen den beiden Hülsenabschnitten 12, 14 liegt ein Fixierungsabschnitt 16, an dem 6 über den Umfang gleichmäßig verteilte pilzartig ausgestaltete Speichenkörper 18 angeordnet sind. Die mit 20 bezeichneten Stege setzen sich auf Seiten des längeren Hülsenabschnitts 12 in Rippen 22 fort, die zum stirnseitigen Ende hin radial leicht verjüngt (siehe Figur 3) in Rippenfortsätzen 24 auslaufen. Zwischen den Rippen 22 ist der längere Hülsenabschnitt 12 dünnwandig ausgebildet.

Aus der Figur 5 ist ersichtlich, dass die Speichenkörper 18, die Rippen 22 und die Rippenfortsätze 24 die Aufgabe übernehmen, die Statorwicklung in genau strukturierter Form zu fixieren.

Der kürzere Hülsenabschnitt 14 ist hohlzylindrisch mit einem Außendurchmesser von etwa 11 mm ausgebildet.

Aufgrund der kleinen Abmessungen des Motors ergibt sich, dass es sich bei dem Spulenkörper 10 um ein hochpräzise zu fertigendes Bauteil handelt, bei dem Kleinstmaße, wie z.B. eine Speichenbreite B20 von 1 mm, eine Rippenstärke S22 von 1,5 mm mit Toleranzbreiten von 0,05 mm exakt eingehalten werden müssen. Aus Gründen der Wirtschaftlichkeit, wird der Spulenkörper als Kunststoff-Spritzgussteil ausgebildet.

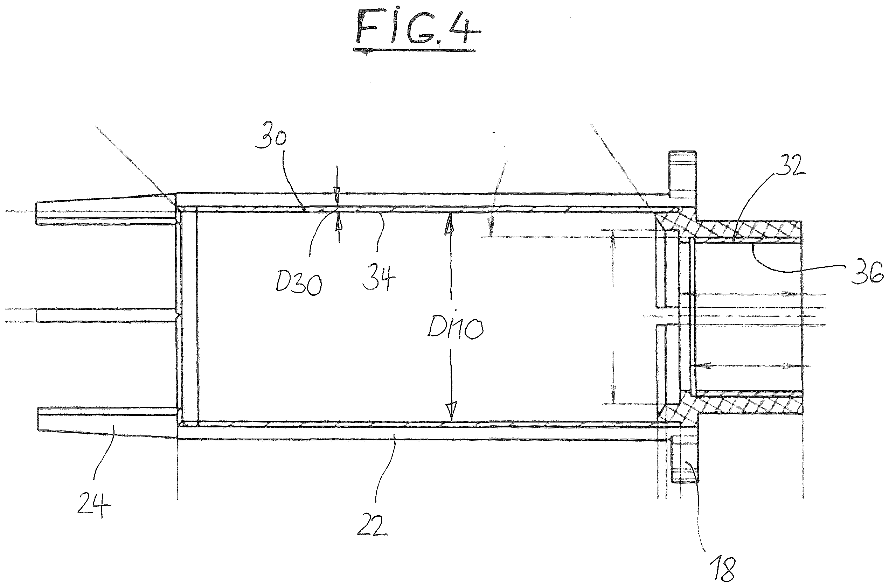

Um die Leistung des Motors weiter anheben zu können, kommt es darauf an, dass der zur Verfügung stehende Bauraum so vollständig wie möglich für die Aufnahme der Statorwicklung - wie in Figur 5 gezeigt - genutzt wird. Um dabei gleichzeitig die Langzeit-Stabilität des Motors zu garantieren zeichnet sich der in den Figuren dargestellte Spulenkörper 10 dadurch aus, dass er als Verbund-Körper ausgebildet ist, bei dem in dem im Spritzgussverfahren hergestellten Kunststoffkörper, der in den Figuren 3 und 4 mit Kreuzschraffur hervorgehoben ist, zumindest ein formstabilisierendes Gerüst-Element durch Umspritzen mit Kunststoff eingebettet ist. Im gezeigten Ausführungsbeispiel handelt es sich um zwei Keramikhülsen 30, 32 unterschiedlichen Durchmessers, bei denen es sich um die anspruchsgemäßen Gerüst-Elemente handelt und die sich über die gesamte Länge des kürzeren Hülsenabschnitts 14 und über beinahe die gesamte Länge des längeren Hülsenabschnitts 12 erstrecken und jeweils im Bereich des Fixierungsabschnitts 16 enden.

Im gezeigten Fall ist als formstabilisierendes Gerüstelement eine Keramikkomponente gewählt, weil das Gerüstelement - wie aus den Figuren 3 und 4 ersichtlich - über weite Strecken die Innenoberfläche 34, 36 des Spulenkörpers10 ausbildet, der dem Rotor gegenüberliegt und deshalb aus einem elektrisch nicht-leitenden Material bestehen soll. Hier bietet sich im Besonderen eine Oxidkeramik an. Die Innenoberfläche 34, 36 des Gerüstelements wird zur Bereitstellung einer möglichst großen Formgenauigkeit auf Maß geschliffen, wodurch es möglich ist, über die gesamte Länge des Stators mit gleichbleibenden, engsten Luftspalten zwischen Rotor und Stator zu arbeiten. Wenn das Gerüstelement allerdings vollständig mit Kunststoff umspritzt ist, können auch andere, die Formstabilität des Spritzgusskörpers erhöhende Materialien, wie z.B. metallische Werkstoffe oder andere keramische Werkstoffe wie z.B. Siliciumcarbid (SiC) oder Cermet-Werkstoffe verwendet werden.

Im gezeigten Ausführungsbeispiel ist der Kunststoffanteil des Spulenkörpers 10 so weit wie möglich reduziert, indem sich zwischen den Rippen 22 des längeren Hülsenabschnitts 12 kein Kunststoff mehr befindet, was aus der Schnittdarstellung gemäß Figur 4 hervorgeht. Dennoch gelingt es, dem Spulenkörper 10 eine ausreichend große Langzeit-Formstabilität zu geben, selbst wenn die Wandstärke D30 der Keramikhülse 30 in Bereich von 0,01 bis 0,03 x DI10 liegt, wobei DI10 den Innendurchmesser des Spulenkörpers 10 bedeutet. Bei einer Dimensionierung gemäß dem gezeigten Ausführungsbeispiel liegt die Wandstärke D30 der Keramikhülse 30 bzw. 32 im Bereich zwischen 0,1 bis 0,5 mm.

Es hat sich gezeigt, dass es mit dem beschriebenen Aufbau gelingt, den Spulenkörper 10 unter Beibehaltung der herkömmlichen Montageschritte für die Anbringung der Statorwicklung und für die Fixierung im Motorgehäuse mit einer bislang nicht erreichten Langzeit-Formstabilität auszustatten, die auch wiederholten Sterilisierungen bzw. Aufbereitungen des Motors standhält, was es erlaubt, den Luftspalt zwischen Spulenkörper und Rotor weiter zu reduzieren.

Selbstverständlich sind Abweichungen von der gezeigten Ausführungsform möglich, ohne den Grundgedanken der Erfindung zu verlassen. Im beschriebenen Fall ist der Spulenkörper sehr komplex mit einer Vielzahl von Passungs- und Funktionsflächen gestaltet, so dass aus Gründen der Wirtschaftlichkeit der Herstellung als Basis ein spritzgegossener Kunststoffkörper 7 verwendet wird. Bei einfacheren geometrischen Formen des Spulenkörpers ist es jedoch auch möglich, und hierin wird eine selbständige Erfindung gesehen, den Spulenkörper vollständig als nicht-leitenden Vollkeramikkörper auszubilden.

Das formstabilisierende Gerüst-Element kann selbstverständlich auch jede andere Form haben, solange es die Funktion erfüllt, die Formstabilität zu steigern. Das Gerüstelement kann auch eine netz- oder Gitterstruktur haben.

Die Erfindung schafft somit einen Spulenkörper für einen Elektromotor, insbesondere einen Hochgeschwindigkeits-Chirurgiemotor, bei dem ein Rotor konzentrisch von dem zumindest eine Statorwicklung tragenden im Wesentlichen hohlzylindrischen Spulenkörper umgeben ist. Um mit vertretbaren Aufwand bei der Aufbringung der Statorwicklung und bei den weiteren Montageschritten die Einhaltung engster Spalte zwischen dem vorzugsweise im Spritzgussverfahren oftmals mit komplexer Formgebung hergestellten Spulenkörper und einem darin laufendem Rotor sicher zu stellen, besteht die Besonderheit darin, dass der Spulenkörper entweder insgesamt von einem nichtleitenden Vollkeramikkörper gebildet oder als Verbund-Körper ausgebildet ist, bei dem in einen vorzugsweise im Spritzgussverfahren hergestellten Kunststoffkörper zumindest ein formstabilisierendes Gerüst-Element durch Umspritzen mit Kunststoff eingebettet ist.

## Patentansprüche

1. Elektromotor, insbesondere Hochgeschwindigkeits-Chirurgiemotor, mit einem Rotor, der konzentrisch von einem zumindest eine Statorwicklung (40) tragenden im Wesentlichen hohlzylindrischen Spulenkörper (10) umgeben ist, wobei der Spulenkörper (10) und die Statorwicklung (40) einen Stator bilden und der Spulenkörper (10) auf seiner Außenseite mit gleichmäßig verteilten Stegen (20) ausgestattet ist, die mit pilzartig ausgestalteten Speichenkörpern (18) versehen sind und die sich in Rippen (22) fortsetzen, die zum stirnseitigen Ende hin in Rippenfortsätzen (24) auslaufen, wobei die pilzartig ausgestalteten Speichenkörper (18), Ripper (22) und Rippenfortsätze (24) dafür vorgesehen und ausgebildet sind, Drähte der Statorwicklung (40) in genau strukturierter Form zu fixieren, wobei zur Bereitstellung des Stators
- zunächst der Spulenkörper (10) mit pilzartig ausgestalteten Speichenkörpern (18) und Rippenfortsätzen (24), die sich zum stirnseitigen Ende hin radial leicht verjüngen, als Verbund-Körper ausgebildet wird, indem zumindest ein formstabilisierendes Gerüst-Element (30, 32) in einen im Spritzgussverfahren hergestellten Kunststoffkörper durch Umspritzen mit Kunststoff eingebettet wird und
- anschließend die Drähte auf und um die Rippen (22) und Rippenfortsätze (24) gewickelt werden.

2. Elektromotor nach Anspruch 1, **dadurch gekennzeichnet, dass** das Gerüst-Element die Form eines Hülsenkörpers (30, 32) hat.

3. Elektromotor nach Anspruch 2, **dadurch gekennzeichnet, dass** der Hülsenkörper (30, 32) aus nichtleitendem Material besteht und zumindest abschnittsweise eine zylindrische Innenoberfläche (34, 36) des Spulenkörpers (10) ausbildet.

4. Elektromotor nach Anspruch 3, **dadurch gekennzeichnet, dass** der Hülsenkörper eine Wandstärke (D30) hat, die im Bereich von 0,01 bis 0,03 x D liegt, wobei D den Innendurchmesser des Spulenkörpers (10) bedeutet.

5. Elektromotor nach Anspruch 4, **dadurch gekennzeichnet, dass** die Wandstärke (D30) des Hülsenkörpers (30, 32) im Bereich zwischen 0,1 bis 0,5 mm liegt.

6. Elektromotor nach einem der Ansprüche 1 bis 5, **dadurch gekennzeichnet, dass** das Gerüst-Element (30, 32) von einer Keramikkomponente, vorzugsweise aus einer Oxidkeramik gebildet ist.

## Claims

1. An electric motor, in particular a high-speed surgical motor, comprising a rotor which is concentrically surrounded by a substantially hollow-cylindrical coil former (10) which carries at least one stator winding (40), wherein the coil former (10) and the stator winding (40) form a stator and the coil former (10) is provided on its outer side with uniformly distributed lands which are provided with mushroom-shaped spoke members (18) and which extend into ribs (22) which terminate towards the front end in rib extensions (24), wherein the mushroom-shaped spoke members (18), ribs (22) and rib extensions (24) are provided and configured for fixing wires of the stator winding (40) in a precisely structured form, wherein for providing the stator
- the coil former (10) with mushroom-shaped spoke members (18) and rib extensions (24), which taper slightly radially towards the front end, is first configured as a composite body in that at least one dimension-stabilizing frame element (30, 32) is embedded by injection molding with plastic in a plastic body which is produced by injection molding, and
- subsequently winding the wires onto and around the ribs (22) and rib extensions (24).

2. The electric motor according to claim 1, **characterized in that** the frame element takes the shape of a sleeve body (30, 32).

3. The electric motor according to claim 2, **characterized in that** the sleeve body (30, 32) consists of non-conductive material and at least in portions forms a cylindrical inner surface (34, 36) of the coil former (10).

4. The electric motor according to claim 3, **characterized in that** the sleeve body has a wall thickness (D30) which is within the range of from 0.01 to 0.03 x D, with D being the inner diameter of the coil former (10).

5. The electric motor according to claim 4, **characterized in that** the wall thickness (D30) of the sleeve body (30, 32) is within the range of from 0.1 to 0.5 mm.

6. The electric motor according to one of the claims 1 to 5, **characterized in that** the frame element (30, 32) is formed by a ceramic component, preferably by oxide ceramics.

## Revendications

1. Moteur électrique, en particulier moteur chirurgical à grande vitesse, avec un rotor qui porte au moins un enroulement de stator (40) et qui est entouré de façon concentrique par un corps de bobine (10) essentiellement cylindrique et creux, dans lequel le corps de bobine (10) et l'enroulement de stator (40) forment un stator et le corps de bobine (10) est aménagé sur son côté extérieur avec des traverses (20) réparties uniformément, lesquelles sont dotées de corps de rayon (18) réalisés en forme de champignon et se prolongent en nervures (22) qui se déroulent en prolongements de nervure (24) en allant vers l'extrémité du côté frontal, dans lequel les corps de rayon (18) réalisés en forme de champignon, les nervures (22) et les prolongements de nervure (24) sont prévus et conçus pour fixer des fils métalliques de l'enroulement de stator (40) en une forme exactement structurée, dans lequel pour la mise à disposition du stator
- d'abord le corps de bobine (10) avec des corps de rayon (18) en forme de champignon et des prolongements de nervure (24) qui s'effilent radialement en allant vers l'extrémité du côté frontal est formé en tant que corps de liaison en ce qu'au moins un élément de châssis (30, 32) stabilisateur de forme est intégré, par enrobage par injection avec une matière plastique, dans un corps en plastique fabriqué dans un procédé de moulage par injection et
- ensuite sont enroulés les fils métalliques sur et autour des nervures (22) et des prolongements de nervure (24).

2. Moteur électrique selon la revendication 1, **caractérisé en ce que** l'élément de châssis présente la forme d'un corps de douille (30, 32).

3. Moteur électrique selon la revendication 2, **caractérisé en ce que** le corps de douille (30, 32) est composé d'un matériau non conducteur et forme au moins par endroits une surface intérieure (34, 36) cylindrique du corps de bobine (10).

4. Moteur électrique selon la revendication 3, **caractérisé en ce que** le corps de douille présente une épaisseur de paroi (D30) qui se situe dans la plage de 0,01 à 0,03 x D, dans lequel D désigne le diamètre intérieur du corps de bobine (10).

5. Moteur électrique selon la revendication 4, **caractérisé en ce que** l'épaisseur de paroi (D30) du corps de douille (30, 32) se situe dans la plage de 0,1 à 0,5 mm.

6. Moteur électrique selon l'une des revendications 1 à 5, **caractérisé en ce que** l'élément de châssis (30, 32) est formé d'une composante en céramique, de préférence une céramique oxydée.
